# EUROPEAN PATENT SPECIFICATION

(11) **EP 2 387 978 B1**
(45) Date of publication and mention of the grant of the patent: **11.06.2014**
(21) Application number: 11382152.4
(22) Date of filing: 16.05.2011
(51) Int. Cl.: A61F 5/08, A61F 5/56

(54) **Removable nasal device**
Entfernbare Nasenvorrichtung
Dispositif nasal amovible

(30) Priority: 17.05.2010 ES 201030730
(43) Date of publication of application: 23.11.2011
(73) Proprietor: Respirfix, S.L., 08192 Sant Quirze del Valles (ES)
(72) Inventor: Yanguas Ezquerro, Jose Maria, 08230 MATADEPERA (ES)
(74) Representative: Sugrañes Patentes y Marcas

(56) References cited:
- WO-A2-2008/091782

## Description

### Technical Field of the Invention

The invention relates to a removable nasal device intended for opening nasal orifices to allow the passage of an air flow.

### Background of the Invention

In order to maintain good health, it is necessary for the lungs to be suitably ventilated by means of breathing. In cases of exerting force, such as while performing physical exercise, it is recommendable to increase the air flow that can pass through the nostrils to improve ventilation and thus improve performance. It is also desirable to increase said air flow in situations such as during sleep times to prevent the so called obstructive sleep apnea syndrome. This sleep disorder is due to repeated incidents of upper airway obstruction or collapse that happens while the affected person sleeps. This causes a collapse, either by means of the reduction (hypopnea) or by means of the complete detention (apnea) of air flow towards the lungs, and it may cause, among other effects, a reduction of blood oxygen levels and an increase of the blood CO₂ level. Said disorder may involve excessive sleepiness during the day due to poor sleep quality affecting intellectual capacity and performance, and it may be the cause of work or traffic accidents. It may also involve respiratory, cardiovascular and psychological disorders, so it must be avoided.

Removable devices intended for opening up nostrils being provided with loops joined by an elastic cord which exert slight pressure on their inner faces for improving the passage of air flow, such as that described in patent ES2186478, are known. However, this device has the drawback that it does not allow being adjusted to all nostril sizes, it is being necessary to provide several sizes in order to be correctly adjusted. Another drawback of said devices is that they are equally deformable along the entire perimeter of the loop, being difficult to reach a compromising solution whereby they are sufficiently rigid so that they do not fall out during their use and at the same time sufficiently deformable to adapt to different nasal cavity morphologies.

Devices such as that described in patent document ES1040491 having open loops joined by an elastic cord are also known. Said device allows reducing the contour of the loops by exerting pressure on them until successfully closing the loop, reducing its perimeter and introducing it in this position into the nostril so that upon recovering its original position, it adapts to the nostril morphology. The operation is made difficult because pressure has to be kept on the loop during the introduction, the device easily being able to be poorly placed. Furthermore, since the loops are open and must be introduced in a closed format, they may pinch the wall of said nostril or the hair therein, being able to cause discomfort and injuries.

Therefore, a main objective of the present invention is to disclose a device which can be adapted for different nostril sizes and solves the drawbacks described above.

### Summary of the Invention

The removable nasal device of the present invention is of the type comprising two closed loops with blunt edges joined by an elastically deformable joining bridge and sized for the tight introduction of each in a respective nostril.

This nasal device is essentially characterized in that at least one of said loops is provided with at least one outer strip portion forming a closed end lobe projecting from said loop which allows adjusting its perimeter when said lobe is removed.

According to another feature of the invention, said loops determine respective oblong figures in plan view, such that they are elastically deformable in their transverse direction and essentially non-elastic in their longitudinal direction, allowing a better introduction and adaptability to the nasal cavity.

In a variant of the invention, the loops are arranged symmetrically, each loop comprising a first longitudinal sector, joined to one another by means of a joining bridge which allows comfortably grasping the device.

According to another feature, each loop comprises a second longitudinal sector that is essentially straight and parallel to its respective first longitudinal sector, the outer strip portion extending continuously between the mentioned first and second longitudinal sectors, forming a lobe which allows increasing the perimeter of each loop.

In another embodiment of interest, the first longitudinal sector of each closed loop is longer than the second longitudinal sector, conferring an oblong shape to the loop in addition to reinforcing its resistance to being bent upon exerting pressure in the longitudinal direction.

According to another feature, the ends of the outer strip portion of the loop are tangent to the first and second longitudinal sectors, whereby the residual burr is minimized upon cutting the strip portions.

According to another feature of the invention, the outer strip portion describes in its central position a curved path that is essentially parallel to the respective loop sector it surrounds, therefore the shape of the contour being completely similar to that of the loop.

In a variant of interest, each loop is provided with a first outer strip portion forming a first lobe projecting in its front part and with a second outer strip portion forming a second lobe projecting in its rear part, both lobes being coplanar with said loop.

According to another feature, the loops, the strip portions and the joining bridge are molded forming a single piece, giving the device greater strength and facilitating its production.

In another variant of the invention, the ends of the strip portion are weakened to facilitate their separation from the associated loop by traverse cutting and thus achieving a clean cut.

In a variant of interest, the joining bridge comprises a strip describing an arch having a curvature greater than 150° in a non-operating position, its ends prolonging in a straight line until joining the central part of the corresponding first longitudinal sector of a loop. The introduction of the device into the nostrils is thus facilitated without needing to previously bend the joining bridge.

In another variant, the ends of the arch widen before joining the central part of the respective first longitudinal sector of each loop, allowing the joining to be more robust.

According to another feature, the direction of each of the ends of the joining bridge forms an angle between 45° and 90° with the plane in which each loop is inscribed. This inclination allows facilitating the introduction of the device, as well as allowing the latter to be better applied against the walls of the nasal cavity.

According to another feature, the central part of the joining bridge, which may coincide with the central part of the arch, is molded forming a concavity suitable for containing a liquid or doughy substance, thus being able to store a small amount of odorous substance, such as an aromatic or medicinal oil.

### Brief Description of the Drawings

The removable nasal device according to the invention is illustrated in the attached drawings by way of non-limiting example. Specifically:
Figure 1 is a plan view of a first variant of the device;
Figure 2 is a front view of the first variant of the device;
Figure 3 is a perspective depiction of the first variant of the device;
Figure 4 is a plan view of a second variant of the device;
Figure 5 is a front view of a nose with the device introduced therein and in the operating position; and
Figure 6 shows the section of a nasal cavity incorporating the device 4, trimmed and introduced in the operating position.

### Detailed Description of the Drawings

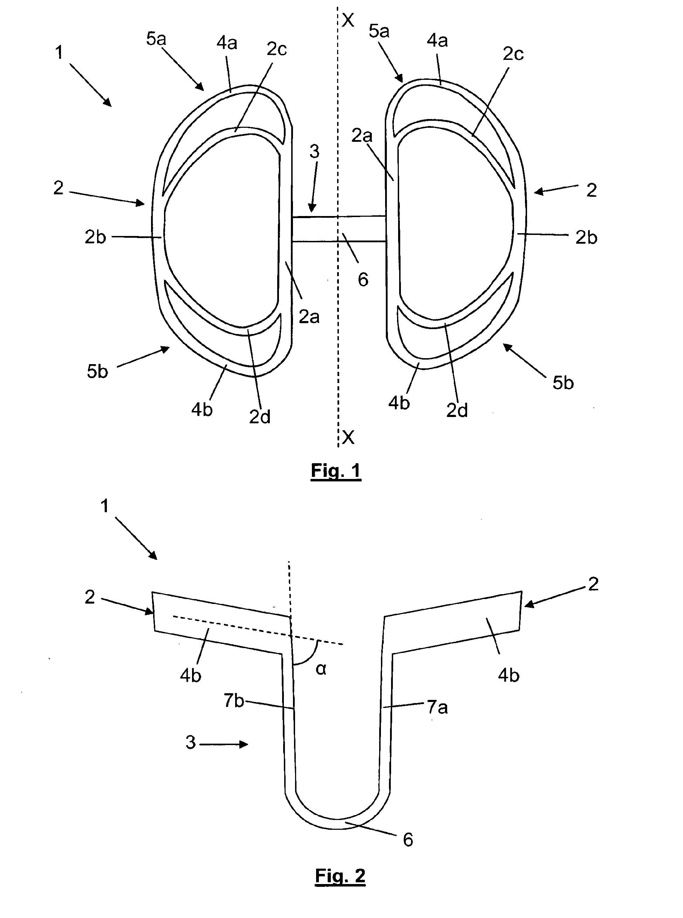

Figures 1 to 3 depict a first variant of the nasal device 1 provided with two loops 2 sized for the tight introduction of each in the nostrils and joined by a joining bridge 3. As can be observed, said loops 2 are symmetrical with respect to the XX plane of section, having the same symmetry as the nostrils.

Each of said loops 2 is provided with first and second outer strip portions 4a, 4b in its front part and in its rear part, respectively, forming a first and second lobe 5a, 5b projecting from and coplanar with its corresponding closed loop 2, increasing the perimeter of each of the loops 2.

As can be observed in Figure 1, each loop 2 comprises first and second longitudinal sectors 2a, 2b that are essentially straight and parallel to one another, from which the outer strip portions 4a, 4b extend, initially prolonging in the same direction as said straight longitudinal sectors 2a, 2b and conferring inelasticity to the loops 2 of the device 1 and, therefore, rigidity in their longitudinal direction, while they are elastically deformable in their transverse direction. This configuration allows the loops to be sufficiently rigid so that they are not deformed when introduced in the nostrils.

Since the first longitudinal sector 2a of each loop 2 is longer than the second essentially straight sector 2b, the loops 2 have the characteristic oblong shape observed in Figure 1, conferring better adaptation to the shape of the nostrils.

The outer strip portions 4a, 4b describing a curved path that is essentially parallel to the respective loop sector 2c, 2d they surround emerge from the ends of the first sector 2a and second longitudinal sector 2b. Said strip portions 4a, 4b form the respective first and second lobes 5a, 5b increasing the perimeter of the respective loops 2, allowing the device 1 to be able to vary its perimeter when one or both of the strip portions 4a, 4b are cut, removing the respective lobes 5a, 5b. Since these are removed by means of cutting each of the strip portions 4a, 4b, it is possible to adjust the perimeter around the loops 2 of the device 1. The device 1 thus adapts to different nostril sizes and morphologies by means of this operation, further enabling the user himself to carry out said operation by means of common cutting elements, such as scissors. To facilitate said cutting operation, it is provided that the ends of the strip portions 4a, 4b have means to aid in cutting, such as weakened areas or having guide lines to facilitate the cutting thereof. Naturally, said means to aid in cutting must be such that they do not allow the strip portions 4a, 4b to come out during daily use of the device 1.

As observed in Figures 1 to 3, the device 1 has a joining bridge 3 comprising an arch 6 having a curvature of 180°, the ends 7a, 7b of which prolong in a straight line, each end widening prior to joining the central part of the first longitudinal sector 2a of each loop 2. Since said ends 7a, 7b widen, certain resistance to twisting is conferred to the joining bridge 3 such that it makes turning the loops 2 after being introduced in the nostrils difficult, thus facilitating the operation of introducing the device 1 in the nostrils. Said joining bridge 3 is also molded forming a single piece together with the rest of the device 1, all contours of the device 1 further being rounded to avoid causing injuries during the insertion and removal operation, as well as during its use.

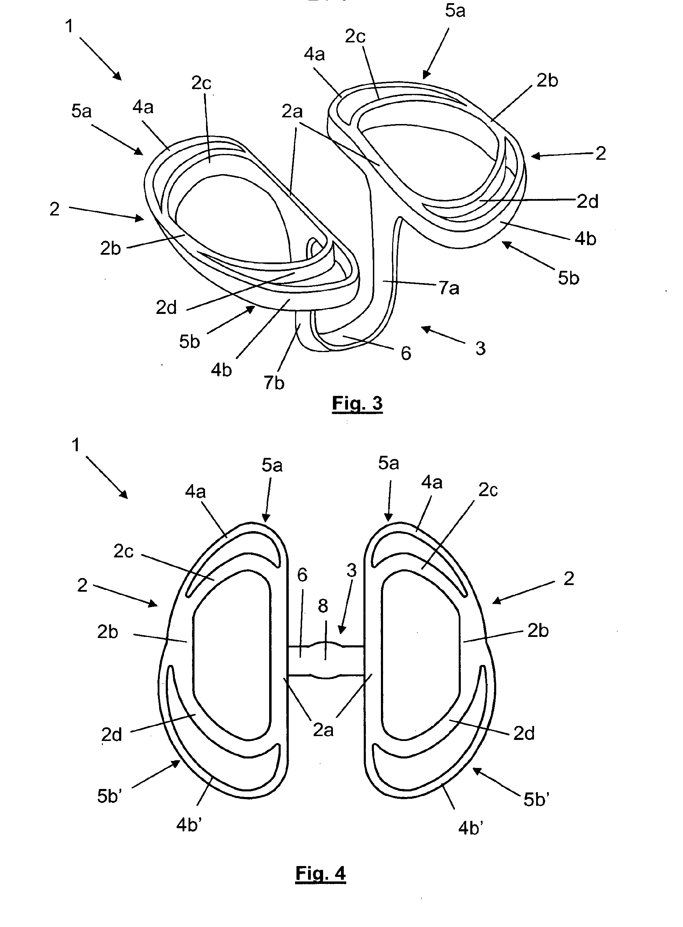

Although each loop 2 only has two strip portions 4a, 4b in the variant depicted in Figures 1 to 3, other variants of the device 1 can have more strip portions allowing the device 1 to be able to adapt to various nostril sizes upon being cut. Although in the variant shown in Figures 1 to 3 the lobes 5a, 5b are symmetrical with respect to the axis which transversely crosses the loop 2, it is possible that this symmetry is not maintained, as shown in the variant of Figure 4 in which the strip portion 4b' projects from the central part of the second longitudinal sector 2b of each loop 2, determining a second larger rear lobe 5b'. This configuration is advantageous since the lower part of the nostrils is usually broader than the upper part.

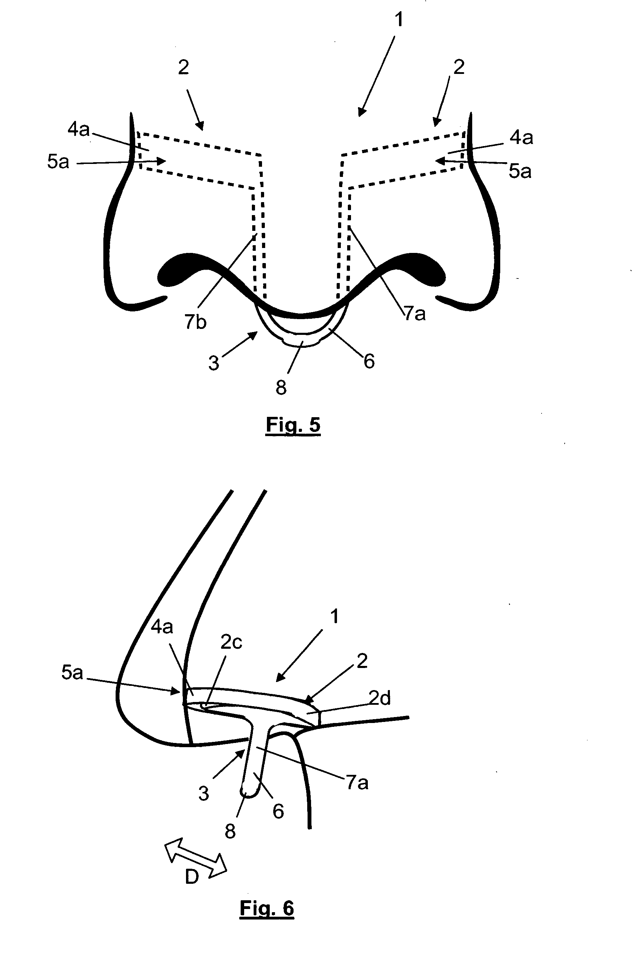

To introduce the device 1 inside the nostrils, the device is advantageously provided with a suitable arch 6 in the joining bridge 3 so that the device 1 can be grasped by the user and pressure thus exerted thereon, introducing the end of the loops 2 in the nostrils, acting as a lever until the loops 2 are introduced in their entirety inside the nostrils, said arch 6 being applied against the nasal septum, thus establishing its operating position, in which the loops 2 are tightly introduced in the nostrils. It is necessary that the loops 2 are arranged in the part of the nostrils coinciding with the nerve parts of the two outer cartilages in said operating position shown in Figures 5 and 6, therefore the ends 7a, 7b of the arch 6 must be correctly sized so that the loops 2 are arranged in said part of the nostrils when the arch 6 is applied against the central septum, preventing the loops 2 from being able to reach upper areas of the nostrils in which they may cause discomfort or injuries.

Upon introducing the device 1 in the nostrils, the loops 2 allow opening the nostrils, exerting pressure both in the longitudinal direction and in the transverse direction which allows expanding each nasal passage, facilitating the passage of air while breathing. The device 1 also has the advantage of offering certain resistance to small frontal impacts and absorbing small side impacts.

To allow the device 1 to be correctly placed inside the nostrils, the direction of each of the ends 7a, 7b of the arch 6 forms an angle α of 62.5°, as can be observed in Figure 2, with the plane in which each loop 2 is inscribed, the loops 2 being applied with certain inclination against the inner walls of the nostrils, as shown in Figure 5. This arrangement improves its grip, preventing the device 1 from coming out during use. However, the outer walls of the loops 2 are straight such that their blunt edges are applied essentially parallel against the inner walls of the nostrils such that they do not cause cuts or injuries, being able to exert the pressure necessary to open the nostrils.

Figure 6 shows the device 1 in the operating position in a section of nostril, in which its first lobe 5a is supported against the front wall of the nostril and the second lobe 5b, 5b' has been trimmed in order to better adapt to the user's nostril. Therefore, the loop sector 2d is what is applied against the nasal cavity. Naturally, it is also possible to only trim the first lobe 5a or both, depending on if it better adapts to the shape of the user's nostrils.

To allow regulating the pressure exerted by the device 1 in the operating position, the user can grasp the device 1 by the arch 6, moving it in one of the directions shown by the arrow D. The inclination of the loops 2 is thus varied and the pressure exerted against the walls of the nostrils is thus increased or decreased, and, therefore, the opening of the nostrils is regulated. This operation is also advantageous to relieve the pain which may be caused by the device 1 if it has been introduced by exerting too much pressure. To allow this regulation, the joining bridge 3 has to be joined in a sufficiently rigid manner to the loops 2 for allowing the loops 2 to change their inclination according to the user's indication upon moving the arch 6 in the direction of the arrow D.

The variant of the device 1 shown in Figures 4 to 6 incorporates a concavity 8 molded in the central part of the arch 6 in the form of a receptacle. This concavity 8 allows retaining a small amount of doughy product, such as an aromatic oil, increasing the well-being of the user.

As observed in Figures 5 and 6, the device 1 is partially hidden inside the nostrils in the operating position, only part of the arch 6 being visible. Although it is possible for the device 1 to be molded in a material having a color similar to that of the skin of the user such that it mimics his skin, it is also possible to use it like a bead, being able to be of a bright color and even have associated costume jewelry and other ornamentations highlighting it.

To remove the device 1 from inside the nostrils, it is only necessary for the user to pull on the arch 6 which is applied against the central septum in the operating phase. The loops 2 are removed from the inside the nostrils by exerting slight pressure, the device 1 being able to be subsequently re-used after washing it simply using water and soap. To favor the correct removal of the device 1, it is provided that the arch 6 can be provided with holding means such as a ring or a cord which allows pulling on the device 1 and thus removing the loops 2 from inside the nostrils with more ease.

To avoid discomforts during use, it is provided that the device 1 can be molded in a suitably soft, biocompatible material with rounded edges.

## Claims

1. A removable nasal device (1) comprising two closed loops (2) with blunt edges joined by an elastically deformable joining bridge (3) and sized for the tight introduction of each in a respective nostril, **characterized in that** at least one of said loops is provided with at least one outer strip portion (4a, 4b, 4b') forming an end side lobe (5a, 5b, 5b') projecting from said loop.

2. The nasal device (1) according to the preceding claim, **characterized in that** said loops (2) determine respective oblong figures in plan view, such that they are elastically deformable in their transverse direction and essentially non-elastic in their longitudinal direction.

3. The nasal device (1) according to any one of the preceding claims, **characterized in that** the loops (2) are arranged symmetrically, each loop comprising a first longitudinal sector (2a), joined to one another by means of the joining bridge (3).

4. The nasal device (1) according to the preceding claim, **characterized in that** each loop (2) comprises a second longitudinal sector (2b) that is essentially straight and parallel to the first longitudinal sector (2a) of the same loop, the outer strip portion (4a, 4b, 4b') extending continuously between the mentioned first and second longitudinal sectors.

5. The nasal device (1) according to the preceding claim, **characterized in that** the first longitudinal sector (2a) of each closed loop (2) is longer than the second longitudinal sector (2b)

6. The nasal device (1) according to any one of claims 4 to 5, **characterized in that** the ends of the outer strip portion (4a, 4b, 4b') of the loop (2) are tangent to the first and second longitudinal sectors (2a, 2b).

7. The nasal device (1) according to any one of the preceding claims, **characterized in that** the outer strip portion (4a, 4b, 4b') describes in its central position a curved path that is essentially parallel to the respective loop sector (2c, 2d) it surrounds.

8. The nasal device (1) according to any one of the preceding claims, **characterized in that** each loop (2) is provided with a first outer strip portion (4a) forming a first lobe (5a) projecting in its front part and a second outer strip portion (4b) forming a second lobe (5b) projecting in its rear part, both lobes being coplanar with said loop.

9. The nasal device (1) according to any one of the preceding claims, **characterized in that** the loops (2), the strip portions (4a, 4b, 4b') and the joining bridge (3) are molded forming a single piece.

10. The nasal device (1) according to any one of the preceding claims, **characterized in that** the ends of the strip portion (4a, 4b, 4b') are weakened to facilitate their separation from the associated loop (2) by transverse cutting.

11. The nasal device (1) according to any one of claims 3 to 10, **characterized in that** the joining bridge (3) comprises a strip describing an arch (6) having a curvature greater than 150°, its ends (7a, 7b) prolonging in a straight line until joining the central part of the corresponding first longitudinal sector (2a) of a loop (2).

12. The nasal device (1) according to the preceding claim, **characterized in that** the ends (7a, 7b) of the arch (6) widen before joining the central part of the respective first longitudinal sector (2a) of each loop (2).

13. The nasal device (1) according to the preceding claim, **characterized in that** the direction of each of the ends (7a, 7b) of the joining bridge (3) forms an angle α between 45° and 90° with the plane in which each loop (2) is inscribed.

14. The nasal device (1) according to any one of the preceding claims, **characterized in that** the central part of the joining bridge (3) is molded forming a concavity (8) suitable for containing a liquid or doughy substance.

## Patentansprüche

1. Entfernbare Nasenvorrichtung (1) umfassend zwei geschlossene Ringe (2) mit stumpfen Kanten, welche durch eine elastisch verformbare Verbindungsbrücke (3) verbunden sind und für die dichte Einführung von jedem in ein jeweiliges Nasenloch dimensioniert sind, **dadurch gekennzeichnet, dass** mindestens einer der genannten Ringe mit mindestens einem äußeren Streifenteil (4a, 4b, 4b') versehen ist, welcher einen seitlichen Endflügel (5a, 5b, 5b') bildet, der von dem genannten Ring herausragt.

2. Nasenvorrichtung (1) nach dem vorhergehenden Anspruch, **dadurch gekennzeichnet, dass** die genannten Ringe (2) jeweilige Längsfiguren in Draufsicht bestimmen, so dass sie in ihrer transversalen Richtung elastisch verformbar und in ihrer longitudinalen Richtung wesentlich unelastisch sind.

3. Nasenvorrichtung (1) nach einem der vorhergehenden Ansprüche, **dadurch gekennzeichnet, dass** die Ringe (2) symmetrisch angeordnet sind, wobei jeder Ring einen ersten longitudinalen Abschnitt (2a) umfasst, die durch die Verbindungsbrücke (3) miteinander verbunden sind.

4. Nasenvorrichtung (1) nach dem vorhergehenden Anspruch, **dadurch gekennzeichnet, dass** jeder Ring (2) einen zweiten longitudinalen Abschnitt (2b) umfasst, welcher wesentlich gerade und parallel zum ersten longitudinalen Abschnitt (2a) des gleichen Rings ist, wobei sich der äußere Streifenteil (4a, 4b, 4b') durchgehend zwischen dem genannten ersten und dem genannten zweiten longitudinalen Abschnitt erstreckt.

5. Nasenvorrichtung (1) nach dem vorhergehenden Anspruch, **dadurch gekennzeichnet, dass** der erste longitudinale Abschnitt (2a) von jedem geschlossenen Ring (2) länger als der zweite longitudinale Abschnitt (2b) ist.

6. Nasenvorrichtung (1) nach einem der Ansprüche 4 bis 5, **dadurch gekennzeichnet, dass** die Enden des äußeren Streifenteils (4a, 4b, 4b') des Rings (2) in Bezug auf den ersten longitudinalen Abschnitt (2a) und den zweiten longitudinalen Abschnitt (2b) tangential sind.

7. Nasenvorrichtung (1) nach einem der vorhergehenden Ansprüche, **dadurch gekennzeichnet, dass** der äußere Streifenteil (4a, 4b, 4b') in seiner zentralen Position einen gebogenen Weg beschreibt, welcher in Bezug auf den jeweiligen Ringabschnitt (2c, 2d), den er umgibt, wesentlich parallel ist.

8. Nasenvorrichtung (1) nach einem der vorhergehenden Ansprüche, **dadurch gekennzeichnet, dass** jeder Ring (2) mit einem ersten äußeren Streifenteil (4a), welcher einen ersten Flügel (5a) bildet, der in seinem vorderen Teil herausragt, und mit einem zweiten äußeren Streifenteil (4b), welcher einen zweiten Flügel (5b) bildet, der in seinem hinteren Teil herausragt, versehen ist, wobei beide Flügel in der gleichen Ebene wie der genannte Ring liegen.

9. Nasenvorrichtung (1) nach einem der vorhergehenden Ansprüche, **dadurch gekennzeichnet, dass** die Ringe (2), die Streifenteile (4a, 4b, 4b') und die Verbindungsbrücke (3) einstückig geformt sind.

10. Nasenvorrichtung (1) nach einem der vorhergehenden Ansprüche, **dadurch gekennzeichnet, dass** die Enden des Streifenteils (4a, 4b, 4b') geschwächt sind, um ihre Trennung von dem dazugehörigen Ring (2) durch transversales Schneiden zu erleichtern.

11. Nasenvorrichtung (1) nach einem der Ansprüche 3 bis 10, **dadurch gekennzeichnet, dass** die Verbindungsbrücke (3) einen Streifen umfasst, welcher einen Bogen (6) beschreibt, der eine Krümmung größer als 150º aufweist, wobei seine Enden (7a, 7b) in einer geraden Linie verlaufen, bis sie sich mit dem zentralen Teil des entsprechenden ersten longitudinalen Abschnitts (2a) eines Rings (2) verbinden.

12. Nasenvorrichtung (1) nach dem vorhergehenden Anspruch, **dadurch gekennzeichnet, dass** die Enden (7a, 7b) des Bogens (6) breiter werden, bevor sie sich mit dem zentralen Teil des jeweiligen ersten longitudinalen Abschnitts (2a) von jedem Ring (2) verbinden.

13. Nasenvorrichtung (1) nach dem vorhergehenden Anspruch, **dadurch gekennzeichnet, dass** die Richtung von jedem der Enden (7a, 7b) der Verbindungsbrücke (3) einen Winkel α zwischen 45º und 90º mit der Ebene, in welcher jeder Ring (2) einbeschrieben ist, bildet.

14. Nasenvorrichtung (1) nach einem der vorhergehenden Ansprüche, **dadurch gekennzeichnet, dass** der zentrale Teil der Verbindungsbrücke (3) so geformt ist, dass er eine Konkavität (8) bildet, die dazu geeignet ist, eine flüssige oder teigige Substanz zu enthalten.

## Revendications

1. Dispositif nasal amovible (1) comprenant deux boucles fermées (2) avec des bords arrondis reliées par un pont de liaison (3) élastiquement déformable et dimensionnées pour l'introduction étanche de chacune dans une narine respective, **caractérisé en ce qu'**au moins une desdites boucles est munie d'au moins une portion de bande extérieure (4a, 4b, 4b') formant un lobe latéral d'extrémité (5a, 5b, 5c) faisant saillie à partir de ladite boucle.

2. Dispositif nasal (1) selon la revendication précédente, **caractérisé en ce que** lesdites boucles (2) déterminent des figures oblongues respectives en vue en plan, de telle sorte qu'elles sont déformables élastiquement dans leur direction transversale et essentiellement non-élastiques dans leur direction longitudinale.

3. Dispositif nasal (1) selon l'une quelconque des revendications précédentes, **caractérisé en ce que** les boucles (2) sont disposées symétriquement, chaque boucle comprenant un premier secteur longitudinal (2a), reliés l'un à l'autre par l'intermédiaire du pont de liaison (3).

4. Dispositif nasal (1) selon la revendication précédente, **caractérisé en ce que** chaque boucle (2) comprend un deuxième secteur longitudinal (2b) qui est sensiblement rectiligne et parallèle au premier secteur longitudinal (2a) de la même boucle, la portion de bande extérieure (4a, 4b, 4b') s'étendant en continu entre les premier et deuxième secteurs longitudinaux mentionnés.

5. Dispositif nasal (1) selon la revendication précédente, **caractérisé en ce que** le premier secteur longitudinal (2a) de chaque boucle fermée (2) est plus long que le deuxième secteur longitudinal (2b).

6. Dispositif nasal (1) selon l'une quelconque des revendications 4 à 5, **caractérisé en ce que** les extrémités de la portion de bande extérieure (4a, 4b, 4b') de la boucle (2) sont tangents au premier et au deuxième secteurs longitudinaux (2a, 2b).

7. Dispositif nasal (1) selon l'une quelconque des revendications précédentes, **caractérisé en ce que** la portion de bande extérieure (4a, 4b, 4b') décrit dans sa position centrale une trajectoire courbe qui est essentiellement parallèle au secteur de boucle (2a, 2b) respective qu'elle entoure.

8. Dispositif nasal (1) selon l'une quelconque des revendications précédentes, **caractérisé en ce que** chaque boucle (2) est pourvue d'une première portion de bande extérieure (4a) formant un premier lobe (5a) faisant saillie dans sa partie avant et une seconde portion de bande extérieure (4b) formant un second lobe (5b) faisant saillie dans sa partie arrière, les deux lobes étant coplanaires avec ladite boucle.

9. Dispositif nasal (1) selon l'une quelconque des revendications précédentes, **caractérisé en ce que** les boucles (2), les portions de bande (4a, 4b, 4b') et le pont de liaison (3) sont moulés en formant une seule pièce.

10. Dispositif nasal (1) selon l'une quelconque des revendications précédentes, **caractérisé en ce que** les extrémités de la portion de bande (4a, 4b, 4b') sont affaiblies pour faciliter leur séparation de la boucle associée (2) par coupe transversale.

11. Dispositif nasal (1) selon l'une quelconque des revendications 3 à 10, **caractérisé en ce que** le pont de liaison (3) comprend une bande qui décrit un arc (6) ayant une courbure supérieure à 150°, ses extrémités (7a, 7b) se prolongeant en ligne droite jusqu'à rejoindre la partie centrale du premier secteur longitudinal (2a) correspondant d'une boucle (2).

12. Dispositif nasal (1) selon la revendication précédente, **caractérisé en ce que** les extrémités (7a, 7b) de l'arc (6) s'élargissent avant de rejoindre la partie centrale du premier secteur longitudinal (2a) respectif d'une boucle (2).

13. Dispositif nasal (1) selon la revendication précédente, **caractérisé en ce que** la direction de chacune des extrémités (7a, 7b) du pont de liaison (3) forme un angle α compris entre 45° et 90° par rapport au plan dans lequel chaque boucle (2) est inscrite.

14. Dispositif nasal (1) selon l'une quelconque des revendications précédentes, **caractérisé en ce que** la partie centrale du pont de liaison (3) est moulée en formant une concavité (8) apte pour contenir une substance liquide ou pâteuse.
